# EUROPEAN PATENT APPLICATION

(11) **EP 3 714 945 A1**
(43) Date of publication of application: **30.09.2020**
(21) Application number: 19166214.7
(22) Date of filing: 29.03.2019
(51) Int. Cl.: A61Q 5/02, A61K 8/60, A61K 8/86, A61Q 19/10, A61K 8/85, C08B 11/04, C08G 63/66, C08K 5/00

(54) **LIQUID THICKENER COMPOSITION COMPRISING POLYALKOXYLATED POLYOLS POLYESTER HAVING GUERBET ACID MOIETIES**

(71) Applicant: Applechem Inc., Parsippany-Troy Hills, NJ 07054 (US)
(72) Inventor: LIN, Samuel Q., Paramus, NJ New Jersey 07652 (US); QIN, Xu, Quincy, MA Massachusetts 02170 (US)
(74) Representative: Wichmann, Hendrik

(57) **Abstract**

The subject application relates to a liquid thickener composition comprising polyalkoxylated polyols polyester having Guerbet acid moieties. Compounds contained herein relate to polyalkoxylated polyol polyester having a viscosity that allows a product to be poured, yet retained on a desired surface to which it is applied. Embodiments of the compounds may be exemplified by the formula:

Q-[(OA)ₙ-OR]ₘ

## Description

### FIELD OF THE EMBODIMENTS

The field of the present invention and its embodiments relate to polyalkoxylated polyols polyester that can generate a viscosity that allows the product to be poured, yet retained on a desired surface to which it is applied.

### BACKGROUND OF THE EMBODIMENTS

Rheology, or the study of the flow of matter, is applied to consumer products, such as shower gel, shampoo, liquid detergent, dishwashing detergent, hand soap, skin care lotion or cream, hair conditioner, hair styling products, etc. to create a particular viscosity profile. Such a profile is critical to a consumer's product preference and eventual purchasing decision.

Consumers will prefer a product with a rheology profile that causes the product to be stable in the container, have a low enough viscosity to pour out of the container easily, and yet be thick enough to apply to the body, hair, or fabric without dripping out of the consumer's hand or the applied surface. Additionally, the product must be stable and maintain a consistent rheology profile during storage in the warehouse, while in transportation, and while on the shelf for potentially many years.

There are a number of commercial thickeners using esters of polyalkoxylated polyols and fatty acids to thicken surfactant-containing preparations. Examples of commercial products include polyethylene glycol 6000 distearate, also known with INCI name of PEG-150 distearate; PEG 120 methyl glucose dioleate and PEG 120 methylglucose trioleate (Glucomate™ DOE 120 and Glucomate™ VLT); PEG-150 Pentaerythrityl Tetrastearate (Crothix™, Crothix™ Liquid, and Versathix™); PEG-150 Polyglyceryl-2 Tristerate (Genapol LT); PEG/PPG-120/10-Trimethlolpropane Trioleate (Arlypon TT). The number of hydrophilic polyalkoxylated arms are two for PEG-150 distearate, three for Arlypon TT, four for Genapol LT and Crothix, Crothix Liquid, and Versathix, and five for Glucomate DOE 120.

US5,192,462 (Gloor et al.) pertains to a thickening agent comprising of tetra ester made of fatty acids and a polyoxyethylene pentaerythritol with four polyethylene glycol arms. Its preferred chemical structure is the PEG-150 Pentaerythrityl Tetrastearate, which is the base for Crothix, Crothix Liquid, and Versathix Liquid.

US7,709,011 and US7,553,495 (both Klug, et al.), pertains to a thickening agent of oxyalkylated polyglycerol esters with fatty acid for surfactant-containing topical preparation as shown below.

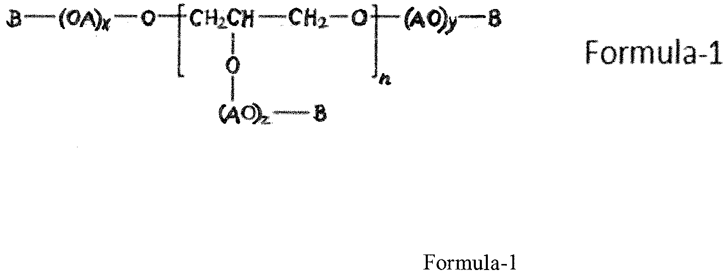

In which A is a group of the formula -C₂H₄- or C₃H₆-, B is a hydrogen or group of the formula -COR, where at least one symbol B is a group of the formula -COR-, R is C₇-C₂₁-Alkyl, C₇-C₂₁-hydroxyalkyl or Alkenyl, n is a number of 1 to 10, preferably from 1.8 to 5, and x, y, z are numbers from 0 to 100, where the sum of x, y, and z is 50 - 250, and preferably from 130-170. This definition of Formula-1 is described at Column 2, lines 24-28.

To those skilled in the art, the number of hydrophilic poly-(ethylene glycol) - arms equals to n+2. When n =10 and x+y+z = 250, the average number of ethylene glycol unit or (x+y+z)/n is [250/ (10+2)] and is about 21 at maximum.

### SUMMARY OF THE EMBODIMENTS

The embodiments of the present invention may be added to a vessel at room temperature or higher temperatures during the manufacturing of cosmetic, dermatological, and pharmaceutical compositions such as the shampoo, shower gels, etc.

The objective of the embodiments of this invention is a flowable liquid comprising a high concentration of the polyalkoxylated polyols polyester of Formula-2, organic carriers, and water.

Q-[(OA)ₙ-OR]ₘ Formula-2

wherein Q is defined as radical of organic polyols compounds, having elements of carbon, hydrogen, oxygen, and nitrogen, and from 6 to 50 carbon atoms, and is saturated or unsaturated, straight, branched or cyclic chain, and independently substituted with from 6 to 25 groups having the formula of [(OA)ₙ-OR]; wherein A is selected from -C₂H₄- or -C₃H₆-; n is from 1-125; R is independently selected from hydrogen or -COR₁; and R₁ is independently selected from C₆-C₃₄-alkyl, C₆-C₂₂-hydroxyalkyl, C₂-C₃₄-alkenyl; wherein R₁ is preferably a stearic moiety, an isostearic moiety, an oleic moiety, a Guerbet moiety having 12 to 32 carbon atoms, or mixtures thereof. "-[(OA)ₙ-OR]" is a radical which is attached to the Q radical. A highly preferred R₁ is an oleic moiety; another highly preferred embodiment is a mixture of isotearic acid, and Guerbet acid. In some embodiments, the Guerbet acid has 12 to 32 carbons, more preferably about 16-32 carbons, more preferably 18-24 carbons, and most preferably 20 carbons, and the average total number of COR₁ (based on the polyalkoxylated polyols polyester conforming to the Formula-2 in the composition) is ≥ 2.5, preferably ≥ 3, and most preferably ≥ 4; and m is an integer selected from 6-25, preferably from 6-12. The n for each hydrophilic poly-(alkylene glycol) - arms can range from 1 - 125, and the average number of is from 25 to 120, prefeably from 30 - 85, and most preferably from 30 to 70.
In one embodiment, the present invention refers to the following items:
1. A liquid thickener composition comprising:
   20%-80% of the polyalkoxylated polyols polyester of the formula:

      Q-[(OA)ₙ-OR]ₘ Formula-2

      Q is a radical of organic polyol compounds, having 6 to 50 carbon atoms, and is saturated or unsaturated, straight, branched or cyclic chain structure, wherein one or more, preferably all, of said carbon atoms are independently substituted with a substituent having the formula of [(OA)ₙ-OR], wherein n = 6 to 25,
      wherein each of the remaining carbon atoms of the 6 to 50 carbon atoms is independently substituted with hydrogen, oxygen (preferably -OH), or nitrogen (preferably -NH₂), particularly preferred hydrogen,
      A is selected from -C₂H₄- or -C₃H₆-,
      R is independently selected from hydrogen or -COR₁,
      wherein the averaged total number of COR₁ in the composition is ≥2,
      R₁ is independently selected from C₆-C₃₄-alkyl, preferably derived from a Guerbet acid; C₆-C₂₂-hydroxyalkyl, preferably derived from a Guerbet acid; C₆ - C₂₂ - alkenyl, preferably derived from a Guerbet acid;
      wherein R₁ is preferably derived from stearic, isostearic, oleic acid, Guerbet acid, or mixtures thereof,
      n is an integer independently selected from 1-125, and
      m is an integer selected from 6-25;
   an organic carrier, selected from the group consisting of: organic solvents, hydrophobic thickeners, or mixtures thereof,
      the organic solvents are selected from the group consisting of: mono- or polyhydric alcohols and their ether, ester, or amide derivatives, glycerol, propylene glycol, 1.3-propanediol, butylene glycol, dipropylene glycol, pentylene glycol, methoxy diglycol, polyglyceryl-3, polyglyceryl-2, glycereth-7, glycereth-26, diglycerol, sorbitol, glucose, methyl glucose, methyl glucamide, glyceryl laurate, glyceryl oleate, glyceryl isostearte, propylene glycol monolaurate, PEG-6 caprylic/capric glyceride, sorbitan carpylate/caprate, sorbitan sesquicaprylate, sorbitane laurate, methyl glucose caprate/caprylate/oleate, PEG-7 glyceryl cocoate, fatty methyl ester ethoxylates, alkylpolyglucosides, decyl glucoside, cocoa glucoside, polysorbate 20, polysorbate 60, and polysorbate 80, alkyoyl methyl glucamide, lauroyl methyl glucamide, capryloyl/caproyl methyl glucamide, and
      the hydrophobic thickeners are selected from the group consisting of: non-ionic alkanolamides, liquid alkanolamides, ethoxylated C₁₀ - C₁₄ alcohols, Laureth-1 to Laureth-5, dimethyl lauramide/myristamide, PPG-2 Hydroxyethyl Cocamide, Cocamide DIPA, lauramide DIPA, soyamide DIPA; and
   water, wherein the ratio of water to organic solvent carrier by weight is from 4:1 to 1:4.
2. The liquid composition of item 1, wherein the C₆-C₃₄-alkyl derived from a Guerbet acid has the following formula: 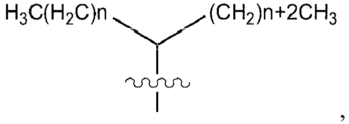 wherein n = 1-120, preferably 3, 5, 7, 9, or 13.
3. The liquid composition of item 1 or 2, wherein the liquid composition further comprises:
   20-80% of the polyalkoxylated polyol polyester of the Formula-2, wherein the Q is a radical of the following polyols compounds:
      sugar alcohols, polyhydric alcohol, or polyalcohol with at least six hydroxyl groups, having the general formula of HOCH₂ (CHOH)ₙ CH₂OH, mannitol, sorbitol, galactitol, fucitol, iditol, inositol, volemitol, isomalt, maltitol, lactitol, maltotritol, and maltotetraitol;
      disaccharide having a glycosidic linkage,
      di-pentaerythritol,
      dextrin with a chemical structure of (C₆H₁₀O₅)ₙ, where n is from 2 to 20,
      dendrimer polyols, and
      polyglyceryls with 3 to 10 glycerin units and 6 or more hydroxyl groups;
   an organic carrier, selected from the group consisting of: organic solvents, hydrophobic thickener, or mixtures thereof,
      the organic solvents are selected from the group consisting of: mono- or polyhydric alcohols and their ether, ester, or amide derivatives, glycerol, propylene glycol, 1.3-propanediol, butylene glycol, dipropylene glycol, pentylene glycol, methoxy diglycol, polyglyceryl-3, polyglyceryl-2, glycereth-7, glycereth-26, diglycerol, sorbitol, glucose, methyl glucose, methyl glucamide, glyceryl laurate, glyceryl oleate, glyceryl isostearte, propylene glycol monolaurate, PEG-6 caprylic/capric glyceride, sorbitan carpylate/caprate, sorbitan sesquicaprylate, sorbitane laurate, methyl glucose caprate/caprylate/oleate, PEG-7 glyceryl cocoate, fatty methyl ester ethoxylates, alkylpolyglucosides, decyl glucoside, cocoa glucoside, polysorbate 20, polysorbate 60, and polysorbate 80, alkyoyl methyl glucamide, lauroyl methyl glucamide, capryloyl/caproyl methyl glucamide, and
      the hydrophobic thickeners are selected from the group consisting of: non-ionic alkanolamides, liquid alkanolamides, ethoxylated C₁₀ - C₁₄ alcohols, Laureth-1 to Laureth-5, dimethyl lauramide/myristamide, PPG-2 Hydroxyethyl Cocamide, Cocamide DIPA, lauramide DIPA, soyamide DIPA; and
   water, wherein the ratio of water to organic solvent carrier by weight is from 4:1 to 1:4.
4. A liquid composition of any preceding item, wherein the liquid thickener composition further comprises:
   20-80% of the polyalkoxylated polyols polyester of the Formula-2, wherein the Q is the radical of the following polyols compounds:
      sugar alcohols, polyhydric alcohol, or polyalcohol with at least six hydroxyl groups, having the general formula of HOCH₂ (CHOH)ₙ CH₂OH, mannitol, sorbitol, galactitol, fucitol, iditol, inositol, volemitol, isomalt, maltitol, lactitol, maltotritol, maltotetraitol, and polyglycitol,
      disaccharide, which is formed from two monosaccharides by dehydration via glycosidic linkage,
      di-pentaerythritol,
      dextrin with a chemical structure of (C₆H₁₀O₅)ₙ, where n is from 2 to 20, and
      dendrimer polyols;
   the organic carrier is selected from the group consisting of: organic solvents, hydrophobic thickeners, or mixtures thereof,
      the organic solvents are selected from the group consisting of: mono- or polyhydric alcohols and their ether, ester, or amide derivatives, glycerol, propylene glycol, 1.3-propanediol, butylene glycol, dipropylene glycol, pentylene glycol, methoxy diglycol, polyglyceryl-3, polyglyceryl-2, glycereth-7, glycereth-26, diglycerol, sorbitol, glucose, methyl glucose, methyl glucamide; glyceryl laurate, glyceryl oleate, glyceryl isostearte, propylene glycol monolaurate, PEG-6 caprylic/capric glyceride, sorbitan carpylate/caprate, sorbitan sesquicaprylate, sorbitane laurate, methyl glucose caprate/caprylate/oleate, PEG-7 glyceryl cocoate, fatty methyl ester ethoxylates, alkylpolyglucosides such as decyl glucoside, cocoa glucoside, polysorbate 20, polysorbate 60, and polysorbate 80, alkyoyl methyl glucamide, lauroyl methyl glucamide, capryloyl/caproyl methyl glucamide, and
      the hydrophobic thickeners are selected from the group consisting of: non-ionic alkanolamides, liquid alkanolamides, ethoxylated C₁₀ - C₁₄ alcohols, Laureth-1 to Laureth-5, and dimethyl lauramide/myristamide, PPG-2 Hydroxyethyl Cocamide, Cocamide DIPA, lauramide DIPA, soyamide DIPA; and
   water, wherein the ratio of water to organic solvent carrier by weight is from 4:1 to 1:4.
5. A liquid composition of any preceding item, wherein the liquid composition further comprises:
   20%-80% of Formula-2, wherein Q is the radical of sorbitol, trehalose, mannitol, di-pentaerythritol, sucrose, lactose, chitobiose, cellobiose, and maltose;
   the organic carrier is selected from the group consisting of: organic solvents, hydrophobic thickeners, or mixtures thereof, wherein
      the organic solvents are selected from the groups consisting of: mono- or polyhydric alcohols and their ether, ester, or amide derivatives, glycerol, propylene glycol, 1.3-propanediol, butylene glycol, dipropylene glycol, pentylene glycol, methoxy diglycol, polyglyceryl-3, polyglyceryl-2, glycereth-7, glycereth-26, diglycerol, sorbitol, glucose, methyl glucose, methyl glucamide, glyceryl laurate, glyceryl oleate, glyceryl isostearte, propylene glycol monolaurate, PEG-6 caprylic/capric glyceride, sorbitan carpylate/caprate, sorbitan sesquicaprylate, sorbitane laurate, methyl glucose caprate/caprylate/oleate, PEG-7 glyceryl cocoate, fatty methyl ester ethoxylates, alkylpolyglucosides, decyl glucoside, cocoa glucoside, polysorbate 20, polysorbate 60, and polysorbate 80, alkyoyl methyl glucamide, lauroyl methyl glucamide, and capryloyl/caproyl methyl glucamide, and
      the hydrophobic thickeners are selected from the groups consisting of: non-ionic alkanolamides, liquid alkanolamides, ethoxylated C₁₀ - C₁₄ alcohols, Laureth-1 to Laureth-5, dimethyl lauramide/myristamide; PPG-2 Hydroxyethyl Cocamide, Cocamide DIPA, lauramide DIPA, soyamide DIPA; and
   water, wherein the ratio of water to organic solvent carrier by weight is from 4:1 to 1:4.
6. A liquid composition of any preceding item, wherein the liquid composition further comprises:
   20%-80% of Formula-2, wherein Q is a radical of sorbitol, trehalose, mannitol, di-pentaerythritol, sucrose, lactose, chitobiose, cellobiose, and maltose;
   the organic carrier or mixtures thereof, selected from the group consisting of alkyl polyglucosides, alkyl methyl glucamide, sorbitan laurate, sorbitan caprylate/caprate, liquid non-ionic alkanolamide surfactants, laureth-1, laureth-2, laureth-3, glyceryl caprylate/caprate, and glyceryl laurate; and
   water, wherein the ratio of water to organic solvent carrier by weight is from 4:1 to 1:4.
7. A liquid composition of any preceding item, wherein the liquid composition further comprises:
   20%-80% of Formula-2, wherein Q is the radical of sorbitol, trehalose, mannitol, di-pentaerythritol, sucrose, lactose, chitobiose, cellobiose, and maltose;
   the organic carrier or mixtures thereof, selected from the groups consisting of the alkanolamides; and
   water, wherein the ratio of water to organic solvent carrier by weight is from 4:1 to 1:4.
8. A cosmetic, dermatological, or pharmaceutical composition, in the form of hair cleansing/treatment formulation, skin and body cleansing/treatment formulations, and other toiletry formulations, comprising the liquid thickener composition of any of the preceding items.
9. A cosmetic, dermatological, or pharmaceutical composition comprising the liquid thickener composition of item 5, in the form of hair cleansing/treatment formulation, skin and body cleansing/treatment formulations, and other toiletry formulations.
10. A cosmetic, dermatological, and pharmaceutical composition of item 7 further comprising water, and
   1% - 50% by weight of surfactants selected from the group consisting of: anionic surfactant, cationic surfactant, non-ionic surfactant, amphoteric surfactant, and mixtures thereof;
   0.1% to 15% of the liquid thickener composition of Formula-2; and
   0.1% to 60% of other ingredients selected from the group consisting of: skin and hair actives, stabilizer, further thickeners, and other customary personal cleansing ingredients.
11. A cosmetic, dermatological, and pharmaceutical composition of item 9, further comprising water, and
   1% - 50% by weight of surfactants selected from the group consisting of: anionic surfactant, cationic surfactant, non-ionic surfactant, amphoteric surfactant, and mixtures thereof;
   0.1% to 15% of the liquid thickener composition of Formula-1; and
   0.1% to 60% of other ingredients selected from the group consisting of: skin and hair actives, stabilizer, further thickeners, and other customary personal cleansing ingredients.
12. A cosmetic, dermatological, or pharmaceutical composition of about 1% to 8% of the liquid composition of any of items 1-7, further comprising:
   about 1% to 25% by weight of a skin and hair active ingredient selected from the group consisting of: UV filters, moisturizers, conditioners, antiseptic agents, deodorant actives, reducing agents for permanent wave products, colorants for coloring hair, anti-aging actives, perfume, petrolatum, vegetable oils, cationic conditioning polymers, and mixtures thereof; and
   water.
13. The composition of any of items 1-7, wherein the Guerbet moiety has 16 to 32 carbons.
14. The composition of any of items 1-7, wherein the Guerbet moiety has 18 to 24 carbons.
15. The composition of any of items 1-7, wherein the Guerbet moiety has 20 carbons.

As an example, Formula-3 is the chemical structure of the polyalkoxylated polyols ester of Formula-2, wherein Q is a radical of the organic polyols compound sorbitol.

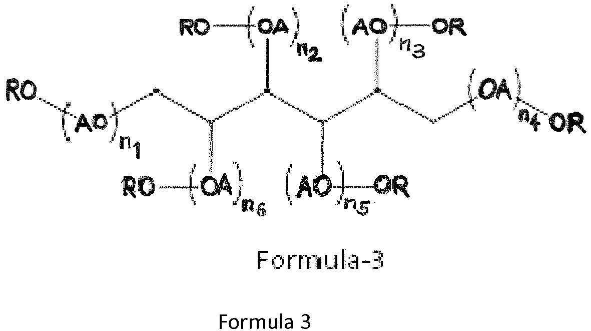

Formula-3 is a reaction product of (a) fatty acid and (b) alkoxylated sorbitol where A is a group of chemical -C₂H₄- or -C₃H₆-; R is hydrogen or a group of formula -COR₁, R₁ is C₆-C₃₄-alkyl, C₆-C₂₂-hydroxyalkyl,C₂ - C₂₂ - Alkenyl, or mixtures thereof, and the sum of R₁ groups is from 3 - 6. The preferred R₁ is derived from stearic, isostearic, oleic, Guerbet acid or mixtures thereof. The most preferred R₁ is oleic, and n₁, n₂, n₃, n₄, n₅ and n₆ are integers from 1 - 120, and the average n is from 25 to 100.

Guerbet acid is a primary carboxylic acid with well-defined twin branching of carbon chain (formula-4). This unique branching structure results in lower melting point, lower viscosity, and better solubility. Examples of Commercial products are ISOCARB® from Sasol, ranging from 12 carbons to 32 carbons. A preferred range of carbons is 16 to 32 carbons, most preferred is 18 to 24 carbons, with the most preferred number of carbons being 20. 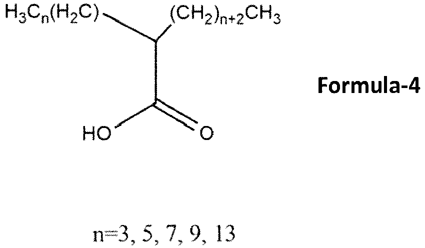 A branched Guerbet acid derived moiety has the following formula: 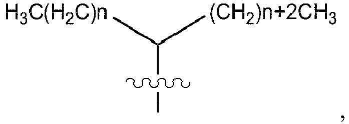 wherein n = 1-120. The symbol " 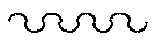 " shows the attachment site of the moiety to the carbon atom of -COR1. In other words "derived from a Guerbet acid" means that R₁ (alkyl, alkenyl or hydroxyalkyl), together with -COOH, forms a Guerbet acid. The flowable liquid thickener of the embodiments of the present invention comprises (a) 20% to 90% of the polyalkoxylated polyols polyester of Formula-2 based on the weight of the total composition, and preferably 30% to 70%; (b) an organic carrier selected from the organic solvents and the liquid hydrophobic thickeners or their mixtures thereof, as defined respectively below; and (c) water. The ratio of water to the organic solvent/hydrophobic thickener is 4:1 to 1:4, and preferably 2:1 to 1:2.

The organic solvents in principle are water-soluble or water-dispersible solvents. They are selected from the groups consisting of mono- or polyhydric alcohols and their ether, ester, or amide derivatives. Examples of alcohols include, but not limited to, propylene glycol, 1.3-propanediol, butylene glycol, dipropylene glycol, pentylene glycol, methoxy diglycol, glycerol, polyglyceryl-2, polyglyceryl-3, glycereth-7, glycereth-26, diglycerol, sorbitol, glucose, methyl glucose, methyl glucamide, etc. Examples of their ester, ether, and amide derivatives include, but not limited to, PEG-6 caprylic/capric glyceride, sorbitan carpylate/caprate, sorbitan sesquicaprylate, sorbitane laurate, polyglyceryl-2 caprylate/caprate, polyglyceryl-3 laurate, PEG-7 glyceryl cocoate; fatty methyl ester ethoxylates; alkylpolyglucosides such as decyl glucoside, cocoa glucoside, etc.; polysorbate 20, polysorbate 60, and polysorbate 80; and alkyoyl methyl glucamide (known as Glucotaine trade name) such as lauroyl methyl glucamide, capryloyl/caproyl methyl glucamide, etc.

Hydrophobic thickeners are thickeners of small molecular weight that increase the viscosity of surfactants in water by affecting the surfactant assembly in water. One class of hydrophobic thickeners is alkanolamides which are condensates of fatty acids or triglycerides with alkanolamines such as monethanol amine, diethanolamine, monoisopropylamines, diisoproplyamines, and other alkoxylated amines. The preferred alkanoamides are the ones which are liquid above 15°C. Examples of the liquid non-ionic alkanolamides include, but not limited to, PPG-2 Hydroxyethyl Cocamide, Cocamide DIPA, lauramide DIPA, soyamide DIPA, Cocamide DEA, Lauramide DEA, etc. Other classes of hydrophobic thickeners include ethoxylated C₁₀-C₁₄ alcohol, and dimethyl lauramide/myristamide.

Another embodiment of this invention is the personal care, cosmetic, dermatological, and pharmaceutical preparations containing the liquid thickener composition of the embodiments of this invention. The liquid thickener composition of the embodiments of this invention is suitable as thickener, rheology modifier, dissolver, and dispersants for aqueous, aqueous/alcoholic, and surfactant-containing preparations; as emulsifiers and suspending agents with a thickening action and bodying action for emulsions and suspensions. These surfactant-containing preparation, emulsions, and suspensions are, for example, shampoo, shower preparations, shower gels, foam baths, facial cleanser, hand soap, bar soap, shaving creams, hair conditioners, deodorants, lotions, creams, ointments, wet wipes, antiperspirants, sunscreens, etc. The embodiments of the invention is also suitable as a thickener and rheology modifier for fabric care products, such as fabric conditioner and liquid laundry detergent.

Based on the finished formulation, the cleansing preparation, and the preparation of the emulsions and suspensions according to this invention comprise preferably 0.05% to 25% by weight, particularly preferably 0.1% to 15% by weight, especially preferably 0.5% to 10% by weight of the liquid thickener of this invention.

The cleansing compositions according to embodiments of this invention can further comprise the following ingredients: all customary anionic, cationic, zwitterionic, nonionic, and amphoteric surfactants; all customary skin and hair benefit actives such as, for examples, cosmetic oils, petrolatum, vegetable oils, hydrogenated vegetable oils, UV filters, proteins, shining agent, anti-aging agents, amino acids, bioactives, humectants, conditioning polymers, silicones, cationic polymers, sucrose polyester, anti-dandruff zinc salt, hydroxyacids, skin lightening agents; all customary stabilizers, such as, for example, silica, 12-hydroxystearic acid, hydrogenated castor oil, ethylene glycol distearate, bentonite and hectorite clay, fatty acid, fatty alcohol; all customary thickeners such as, for example, hydroxyethyl cellulose, xanthan gum, polyacrylate, modified or non-modified starch, etc.; all customary dye, coloring agent, pearlizer, perfume, chelator, solvents, humectants, salt, etc.

The total amount of the surfactants used in the embodiments of this invention can, based on the finished composition, be between 5% and 70% by weight, preferably between 10% and 40% by weight, and most preferably between 12% and 35%.

### Definitions

As used above, and throughout this disclosure, the following terms, unless otherwise indicated, shall be understood to have the following meanings. If a definition is missing, convention definition as known to one skilled in the art controls.

As used herein, the terms "including," "containing," and "comprising" are used in their open, non-limiting sense.

As used herein, the singular forms "a," "an," and "the" include plural referents unless the context clearly dictates otherwise.

To provide a more concise description, some of the quantitative expressions given herein are not qualified with the term "about". It is understood that, whether the term "about" is used explicitly or not, every quantity given herein is meant to refer to the actual given value, and it is also meant to refer to the approximation to such given value that would reasonably be inferred based on the ordinary skill in the art, including equivalents and approximations due to the experimental and/or measurement conditions for such given value. Whenever a yield is given as a percentage, such yield refers to a mass of the entity for which the yield is given with respect to the maximum amount of the same entity that could be obtained under the particular stoichiometric conditions. Concentrations that are given as percentages refer to mass ratios, unless indicated differently.

As used herein, "alkyl" means a straight chain or branched saturated chain having from 1 to 30 or more carbon atoms. An alkyl group can be unsubstituted or substituted. Alkyl groups containing three or more carbon atoms may be straight, branched, or cyclized.

As used herein, an "alkenyl" includes an unbranched or branched hydrocarbon chain having one or more double bonds therein and having from 1 to 30 or more carbon atoms. The double bond of an alkenyl group can be unconjugated or conjugated to another unsaturated group. An alkenyl group can be unsubstituted or substituted.

The term "hydroxyl" means an OH group;

The term "hydroxyalkyl" means an alkyl group as defined above, where the alkyl group has an OH group disposed thereon.

The term "alkoxy" or "alkoxylated" as used herein includes -O-(alkyl), wherein alkyl is defined above.

As used herein, the term "composition" is intended to encompass a product comprising the specified ingredients in the specified amounts, as well as any product which results, directly or indirectly, from combination of the specified ingredients in the specified amounts.

The term "amino" as used herein means a substituent containing at least one nitrogen atom.

As used herein, the term "substituted" means that the specified group or moiety bears one or more suitable substituents wherein the substituents may connect to the specified group or moiety at one or more positions.

As used herein, the term "unsubstituted" means that the specified group bears no substituents.

Any atom that is represented herein with an unsatisfied or unspecified valence is assumed to have the sufficient number of hydrogen atoms to satisfy the atom's valence. For example, "substituted with oxygen" or "substituted with nitrogen" means that the substituent is oxygen bonded via one bond with one hydrogen (-OH) or via two bonds without hydrogen (=O) and nitrogen bonded via one bond with two hydrogens (-NH2) or bonded via two bonds with one hydrogen (=NH).

### DETAILED DESCRIPTION OF THE EMBODIMENTS OF THE INVENTION

The objective of the embodiments of this invention is an easy-to use flowable liquid thickener comprising a high concentration of the polyalkoxylated polyols polyester of Formula-2, organic carriers, and water. This liquid composition is very simple and easy to add to the vessel at room temperature or higher temperatures during the manufacturing of cosmetic, dermatological, and pharmaceutical compositions such as the shampoo, shower gels, etc.

The polyalkoxylated polyols polyester (Formula-2) of this invention is prepared by one or more reaction stages: alkoxylation of Polyols compound, followed by esterification with fatty acids.

Q-[(OA)ₙ-OR]ₘ Formula-2

The polyalkoxylated polyols are prepared by the alkoxylation of the polyols compounds, having 6 to 20 hydroxyl groups, with ethylene oxide or propylene oxide at 130°C - 200°C after drying the mixture of the polyols compound and a base catalyst such as KOH, NaOH, or calcium metal at 100°C -200°C under vacuum. The alkylene oxides are metered into the reactor under pressure over the course of 10-20 hours. Ethylene oxide, propylene oxide, or a mixture of ethylene oxide and propylene oxide can be used, resulting in primary -OH group, secondary - OH group, or mixtures thereof.

The polyols compounds of this invention can be natural polyols or synthetic polyols of having ≥ six hydroxyls, and their examples are shown (but not limited to) from the following classes of compounds:
1. Sugar alcohols, also called polyhydric alcohol or polyalcohol with at least six hydroxyl groups, having the general formula of HOCH₂ (CHOH)ₙ CH₂OH. Examples include, but not limited to, mannitol, sorbitol, galactitol, fucitol, iditol, inositol, volemitol, isomalt, maltitol, lactitol, maltotritol, maltotetraitol, and polyglycitol.
2. Disaccharide, which is formed from two monosaccharides by dehydration via glycosidic linkage. Examples include but not limited to, trehalose, sucrose, lactose, maltose, etc. They contain six or more hydroxyl groups.
3. Di-Pentaerythritol
4. Dextrin with a chemical structure of (C₆H₁₀O₅)ₙ, where n is from 2 to 20.
5. Dendrimer polyols. For examples, Bolton®H2004, H2003, and H20 have 6, 12, and 16 terminal hydroxyls, respectively, Bolton® is the trade name of Perstorp Inc.
6. Polyglyceryls with 3 to 10 glycerin units, with six or more hydroxyl groups.
After the reaction, each hydroxyl group of the starting polyols compounds will grow to a hydrophilic poly-(alkylene glycol) arm. The length of all arms may be the same or different, depending on the reaction conditions.

The next reaction is an esterification reaction between the alkoxylated polyols compounds and the fatty acid, so that some or all of the hydrophilic poly-(alkylene glycol) arms are capped with fatty acid ester. The reaction is carried out between 120°C - 250°C with or without the catalyst until the desired acid number or the degree of esterification is achieved. The preferred method is to use the esterification catalysts such as alkylbenzenesulfonic acid, methansulfonic acid, oranotin catalyst, oranotitanate catalyst, etc. The preferred mole ratio of fatty acid to the alkoxylated polyols compounds is to form esters of 30% - 100% of the hydrophilic poly-(alkylene glycol) arms. For example, when the starting polyols compounds is sorbitol which will lead to six poly-(alkylene glycol) arms per sorbitol molecule, the mole ratio would be 2.4 to 6, leading to Formula-3 Structure. When it is trehalose, which will lead to eight poly-(alkylene glycol) arms, the mole ratio is 3.2 to 8. The most preferred one is to form more than at least four fatty ester capped arms.

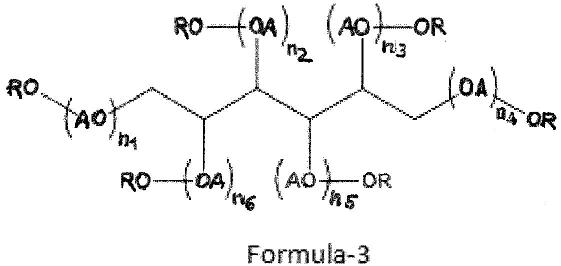

The Q of Formula-2 is defined as the radical of organic polyols compounds, wherein the radical structure is derived from the polyol by removal of one or more, preferably all of, the respective OH-groups, having elements of carbon, hydrogen, oxygen, and nitrogen, and from 6 to 50 carbon atoms, and is saturated or unsaturated, straight, branched or cyclic chain, and independently substituted with from 6 to 25 groups having the formula of [(OA)ₙ-OR]. Wherein A is selected from -C₂H₄- or -C₃H₆-; and the n is from 1-125; R is independently selected from hydrogen or -COR₁; and R₁ is independently selected from C₆-C₃₄-alkyl, C₆-C₂₂-hydroxyalkyl, C₆ - C₂₂ - alkenyl; wherein R₁ is preferably derived from stearic, isostearic, oleic, Guerbet acid, or mixtures thereof and preferred ones are oleic or Guerbet, or a combination of Guerbet and isostearic, and the averaged total number of -COR₁ is ≥ 2.5, preferably ≥ 3.5, and most preferably ≥ 4. The n for the hydrophilic poly-(alkylene glycol) - arm is integers from 1 - 125, and may be the same or different for all arms. The averaged number of n per hydrophilic poly-(alkylene glycol) arm is from 25 to 120, preferably from 30 to 85, and most preferably from 30 to 70.

Guerbet acid is a primary carboxylic acid with well-defined twin branching of carbon chain (formula-4). This unique branching structure results in lower melting point, lower viscosity, and better solubility. A preferred range of carbons is 16 to 32 carbons, most preferred is 18 to 24 carbons, with the most preferred number of carbons being 20.

The flowable liquid thickener of the embodiments of the present invention comprises (a) 20%-90% of the polyalkoxylated polyols polyester of Formula-2 based on the weight of the total composition, and preferably 30% - 70%; (b) an organic carrier selected from the organic solvents and the liquid hydrophobic thickeners or their mixtures thereof, as defined respectively below; and (c) water. The ratio of water to the organic solvent/hydrophobic thickener is 4:1 to 1:4, and preferably 2:1 to 1:2.

The organic solvents in principle are water-soluble or water-dispersible solvents. They are selected from the groups consisted of mono- or polyhydric alcohols and their ether, ester, or amide derivatives. Examples of alcohols include, but not limited to, propylene glycol, 1.3-propanediol, butylene glycol, dipropylene glycol, pentylene glycol, methoxy diglycol, glycerol, polyglyceryl-2, polyglyceryl-3, glycereth-7, glycereth-26, diglycerol, sorbitol, glucose, methyl glucose, methyl glucamide, etc. Examples of their ester, ether, and amide derivatives include, but not limited to, PEG-6 caprylic/capric glyceride, sorbitan carpylate/caprate, sorbitan sesquicaprylate, sorbitane laurate, polyglyceryl-2 caprylate/caprate, polyglyceryl-3 laurate, PEG-7 glyceryl cocoate; fatty methyl ester ethoxylates; alkylpolyglucosides such as decyl glucoside, cocoa glucoside, etc.; polysorbate 20, polysorbate 60, and polysorbate 80; and alkyoyl methyl glucamide (known as Glucotaine trade name) such as lauroyl methyl glucamide, capryloyl/caproyl methyl glucamide; etc.

Hydrophobic thickeners are thickeners of small molecular weight that increase the viscosity of surfactants in water by affecting the surfactant assembly in water. One class of hydrophobic thickeners is alkanolamides which are condensates of fatty acids or triglycerides with alkanolamines such as monethanol amine, diethanolamine, monoisopropylamines, diisoproplyamines, and other alkoxylated amines. The preferred alkanoamides are the ones which are liquid above 15°C. Examples of the liquid non-ionic alkanolamides include, but not limited to, PPG-2 Hydroxyethyl Cocamide, Cocamide DIPA, lauramide DIPA, soyamide DIPA, Cocamide DEA, Lauramide DEA, etc. Other classes of hydrophobic thickeners include ethoxylated C₁₀-C₁₄ alcohol, and dimethyl lauramide/myristamide. NINOL® CAA from Stepan is a commercial example of the dimethyl lauramide/myristamide class.

Another embodiment of this invention is the cosmetic and pharmaceutical preparations containing the flowable liquid thickener composition. The liquid composition of the embodiments of this invention is suitable as thickener, rheology modifier, dissolver, and dispersants for aqueous, aqueous/alcoholic, and surfactant-containing preparations; as emulsifiers and suspending agents with a thickening action and bodying action for emulsions and suspensions. These surfactant-containing preparation, emulsions, and suspensions are, for example, shampoo, shower preparations, shower gels, foam baths, facial cleanser, hand soap, bar soap, shaving creams, hair conditioners, deodorants, lotions, creams, ointments, wet wipes, antiperspirants, sunscreens, etc.

Based on the finished formulation, the cleansing preparation, the preparations of emulsions and suspensions according to this invention comprise preferably 0.05% to 25% by weight, particularly preferably 0.1% to 15% by weight, especially preferably 0.5% to 10% by weight of the easy-to-use liquid thickener composition of this invention containing the polyalkoxylated polyols polyester of Formula-2.

The cleansing compositions according to embodiments of this invention can further comprise the following components: all customary anionic, cationic, zwitterionic, nonionic, and amphoteric surfactants; skin and hair benefit actives such as , for examples, cosmetic oils, petrolatum, vegetable oils, hydrogenated vegetable oils, UV filters, proteins, shining agent, anti-aging agents, amino acids, bioactives, humectants, conditioning polymers, silicones, cationic polymers, sucrose polyester, anti-dandruff zinc salt, hydroxyacids, skin lightening agents, etc.; Stabilizers, such as, for example, silica, 12-hydroxystearic acid, hydrogenated castor oil, ethylene glycol distearate, bentonite and hectorite clay, fatty acid, fatty alcohol, etc.; other thickeners such as, for example, hydroxyethyl cellulose, xanthan gum, polyacrylate, modified or non-modified starch, etc.; and polyethylene glycols. The cleansing compositions can be in the forms of liquid, paste, gels, or solid, and can be for personal cleansing, fabric cleansing, and hard surface cleansing.

The total amount of the surfactants used in the composition of this invention can, based on the finished composition, be between 5% and 70% by weight, preferably between 10% and 40% by weight, and most preferably between 12% and 35%.

Each of these components as well as preferred and optional components in the cleansing compositions is described below.

### A. Detersive surfactants

The customary detersive surfactants may be selected from anionic, cationic, non-ionic, amphoteric/zwitterionic surfactants, or mixtures thereof. The details of these customary detersive surfactants are cited in many prior, such as US 7,659,235 B2; 8,361,450 B2; 8,802,607B2; 3,929,678; 2,528,378 all of which are incorporated by reference; and McCutcheon's, Emulsifiers and Detergents, 1989 Annual, published by M.C. Publishing Co. Anionic surfactants may include alkyl sulfate or alkyl ether sulfate (including alkyl glyceryl ether sulfate). They may also include the sulfate-free anionic surfactants as illustrated below.

Aliphatic sulfonate class, including, but not limited to, a primary alkane (e.g. C₈-C₂₂) sulfonate, primary alkane disulfonate, C₈-C₂₂ alkene sulfonate, alkyl glyceryl ether sulfonate, aromatic alkyl sulfonate, or C₈-C₂₂ Hydroxyalkane sulfonate.

Alkyl sulfosuccinates (including mono- and dialkl, e.g. C₆-C₂₂ sulfosuccinates), alkyl and acyl taurate, alkyl and acyl glycinates, alkyl sulfoacetate, alkyl phosphates, alkyl phosphate ester, alkyoxy alkyl phosphate esters, and acyl lactates, C₈-C₂₂ monoalkyl succinates and maletes. Fatty acyl isethionates, which are typically prepared by the reaction of an isethionates salts such as alkali metal isethionates and an aliphatic fatty acids of 8-20 carbon atoms. Commercial products of fatty acyl isethionates, for examples, are DEFI, Hostapon SCI-78C, Jordapon CI prill, YA-SCI-85, Iselux LQ-CLR-SB, etc. Anionic carboxylate surfactants with the formula of R-(CH₂CH₂O)ₙCO₂M, wherein R is C₈-C₂₀ alkyl; n is 1 to 20; and M is a positive ion(s) such as sodium, potassium, etc. Another class of anionic surfactants is soap or the salts of fatty acids. Sulfonate derivatives of alkyl polyglucoside; include for example, sodium laurylglucosides, hydroxypropylsulfonate, and sodium decylglucosides and hydroxypropylsulfonate.

Other sulfate-free mild surfactants are the class of alkanoyl surfactants prepared from the amino acids. The alkyl group is C₈ to C₂₀, preferably C₁₂ to C₁₆ alkyl group. This class of surfactants may include, for examples, alkanoyl sarcosinates, alkanoyl glycinate, and alkanoyl glutamate. The commercial products, for examples, are Amisoft®, Amilite® of Ajinamoto, Eversoft of Sino Lion, etc.

The preferred anionic surfactants are the sulfate-free mild surfactants and their mixtures thereof. The proportion by weight of the anionic surfactants in the composition according to this invention is in the range of 5% to 35% by weight, preferably 10% to 25% by weight.

Amphoteric or zwitterionic surfactants are the surfactants with both of positive and negative charges. They can be broadly described as derivatives of aliphatic quaternary ammonium, phosphnium, sulfonium compounds, in which the aliphatic radicals can be straight or branched chain, and wherein one of the aliphatic radicals is from C₈ to C₁₈ carbon atoms, and one contains an anionic group, e.g., carboxyl, sulfonate, sulfate, phosphate, or phosphonate. Examples may include the customary betaine, such as N-alkyl-N, N-dimethyl ammonium glycinates, coco-amidopropyl betaine; C₁₂-C₁₈-alkyldimethyl-sulfopropylbetain, and amine oxides. The proportion of the amphoteric surfactants in the composition according to this invention is 0.5% to 30% by weight, and preferably 1% to 15% by weight.

The non-ionic surfactants may include the alkoxylated aliphatic alcohol, acids, amides or alkyl phenol; long chain tertiary amine oxide; long chain tertiary phosphine oxides; dialkyl sulphoxides; sugar amides, such as described in US 5,389,279 and 5,009,814; alkyl polyglucoside as described in US 4,565,647 and 3,723,325 all of which are incorporated by reference. The preferred non-ionic surfactants are alkyl polyglucoside and alkyl polyglucamide. Examples of commercial products may include Plantaren® series of BASF, Ecosense ®Series of Dow Chemicals, Gluco Tain® of Clariant, and Poly Suga Mulse of Colonial Chemicals. The proportion of the non-ionic surfactants according to this invention is in the range of 1% to 20% by weight, and preferably of 1% to 15%.

Cationic surfactants are the surfactants with positive charge groups. The suitable cationic surfactants may include quaternary ammonium salts, such as di (C₁₀-C₂₄)-alkyldimethylammonium chloride, (C₁₀-C₂₄)-alkyltrimethylammonium chloride or sulfate, and N-acylaminoethyl-N, N-diethyl-N-methylammonium chloride. Other customary cationic surfactants are described in reference of US 8,470,305 and 8,470,305 all of which are incorporated by reference. The proportion by weight of cationic surfactants in the composition according to this invention is in the range of 1% to 10%, and preferably 1% to 7% by weight.

### B. Liquid Crystal Inducers and Modifiers

Liquid crystal inducers are small non-ionic molecules. They are believed to be solubilized in the mixtures of surfactants, and to change the packing of surfactant micelles to larger structure aggregates of different shape and size, such as lamellar liquid structures or vesicles, rod and cubic liquid crystals. The alternative name for the liquid crystal inducers is hydrophobic thickeners. They include the class of alkanoamides, alkylamineoxides or mixtures thereof. Examples of this class include mono- and di-ethanolamides, isopropanolamides of fatty acids of 10-20 carbon atoms, PPG-hydroxyethyl cocamides and alkylamineoxides of 10-20 carbon atoms. Another class of chemicals in the liquid crystal inducers is alkoxylated alkyl alcohols of 8 - 18 carbon atoms, preferably 8-12 carbon atoms and 1-4 ethylene oxide units.

Liquid crystal modifiers include fatty acid and fatty alcohol of 8-20 carbon atom, and aliphatic hydrocarbons of less than molecular weight of 400 g/mole. It is believed that they modify the size and shape of the liquid crystals. US 7,655,607B2 (incorporated by reference) is a reference for the range and examples of the liquid crystal inducers and modifiers.

### C. Skin and Hair Benefit Actives

These benefit actives may be water-soluble, water-insoluble, or water dispersible. The water-soluble actives may include, but not limited to, polyols such as glycerin, diglycerin, sorbitol, propylene glycol, propanediol, patenol, and sugar; alpha-hydroxy acids and its salts as well as low molecular weight polyethylene glycols. Water-insoluble and water-dispersible skin and hair benefit actives include, but are not limited to, petrolatum, silicones, vegetable oils, essential oils, emollients, hydrocarbon oils, fatty esters, cationic polymers, oils of high refractive index for shinning, anti-dandruff agents, proteins/protein derivatives, etc. These non water-soluble benefit agents normally exist as emulsion or stripes in the composition. Non-limiting examples in US 7,262,158 are incorporated herein by reference. Other miscellaneous skin and hair benefit actives may include vitamins, lipids (sucrose esters, lanoline, cholesterol, etc.), liposome, essential fatty acids, butters, minerals, anti-microbial, anti-acne, oil control agents, astringents, oil control-agents, scrub and exfoliating particles, essential oils, sunscreens, styling aid, dye, perfume, cyclodextrin/perfume complex, anti-wrinkle actives (amino acids and their derivatives such as N-acetyl-L-cystein), thiols, anti-cellulite agents (caffeine, theophylline, etc.), tanning actives, skin lightening actives, skin soothing agents (such as bisabolol, aloe vera, dipotassium glycyrrhizinate, etc.).

Cationic water soluble/or dispersible polymers are very useful for the compositions according to embodiments of this invention as conditioning actives or deposition aids. The suitable cationic polymers for the compositions according to this invention have the cationic charge density in the range of 0.2 - 8 meq/g and the molecular weight range of 1,000 to 3 million. Their cationic groups are nitrogen-containing moieties such as quaternary ammonium or cationic protonated amino moieties which can be primary, secondary, and tertiary amines. Non-limiting examples of the cationic polymers are described in the CTFA Cosmetic Ingredient Dictionary, 3rd edition, edited by Estrin, Crosley, and Haynes, and US 8470,305, and US 8,105,994 all of which are incorporated by reference.

Non-limiting examples may include copolymers of vinyl monomers having cationic protonated amines or quaternary ammonium functionalities with water-soluble spacer monomers such as acrylamide, methacrylamide, alkyl and dialkyl acrylamides, vinyl pyrrolidone, vinyl caprolactone, etc. Non-limiting specific examples are Polyquaternium-11, -16, -7, -6, -22, -47, - 39. Other suitable cationic polymers include polysaccharide polymers such as cationic cellulose derivatives, cationic starch derivatives, cationic guar gum derivatives, etc. Non-limiting examples include the following trade name products: Jaguar® from Rhone Poulenc, Aqua® and N-Hance polymer from Aqualon, UCARE Polymer from Dow Chemical, MerQuat from Nalco, Galactasol from Henkel, etc.

### D. Stabilizers and Further Thickeners

The stabilizers (or structuring systems) are used to form a crystalline stabilizing network in the composition, preventing the droplets of the lipophilic benefit agents from coalescing and phase separation in the product. Non-limiting examples include a hydroxyl-containing fatty acids, fatty ester, or fatty soap water-insoluble wax-like substance such as 12-hydroxystearic acid, 9,10-dihydroxystearic acid, tri-9,10-dihydroxystearin, and tri-12-hydroxystearin. Other classes of stabilizers are the C₁₀₋₂₂ ethylene glycol fatty acid ester, fumed silica, precipitated silica, smectite clay, etc. Other customary stabilizer examples are disclosed in US 6,194,363 and US 9,138,428 all of which are incorporated by reference. Another class of stabilizer is a gel-network of fatty amphiphiles such as stearic acid and behenyltrimethylammonium chloride, as disclosed in US 8,470,305 incorporated by reference. Another class of stabilizer is a blend of non-modified and modified starch and fatty acid as disclosed in US 6,906,016 incorporated by reference.

Further thickeners for stabilizing the composition and for modifying the viscosity of the composition, according to this invention, are polymers. Non-limiting examples include carbohydrate gums such as cellulose gum, microcrystalline cellulose, cellulose gel, hydroxyethyl cellulose, hydroxypropyl cellulose, sodium carboxymethylcellulose, methyl /propyl cellulose, guar gum, gum karaya, gum tragacanth, gum arabic, gum acacia, gum agar, xanthan gum, and mixtures thereof; modified and non-modified starch granules with gelatinization temperature between 30°C - 85°C, and pregelatinized cold water soluble starch. Further non-limiting examples include the class of hydrophobic associative, cross-linked, alkali swellable acrylate polymers, comprising acidic monomers and associative monomers having hydrophobic end groups, as disclosed in US 9,161,899 (incorporated by reference). Non-limiting commercial examples are Carbopol Aqua SF-1 of Lubrizol, Stabylen 30 of 3V Sigma S.P.A. Aqupec series of Sumitomo Seika of Japan.

Suitable further thickeners may include salt such as sodium chloride and sodium sulfate; cellulose derivatives, such as hydroxyethylcellulose; xanthan gum, guar gum; starch and starch derivatives; carboxyvinyl polymers, such as Carbopol® 940; Polyacrylate emulsions, such as Carbopol®Aqua SF-1 polymer; polyethylene glycol; and polyvinyl alcohol.

The preparation of the emulsions and suspension, according this invention, comprises water; oils; emulsifiers; preferably 0.05% to 20% by weight, particularly preferably 0.1% to 10% by weight, especially preferably 0.5% to 5% by weight of the easy-to-use liquid composition of Formula-1; and other customary ingredients for skin care, hair care, and body care. The nonaqueous parts of the emulsion ranges normally from 2% to 85%, and preferably from 5% to 45%. The oils include, but not limited to, cosmetic oils of refined vegetable oils, refined synthetic or fermented hydrocarbon oils, silicone oils, and synthetic ester oils.

The following non-limiting examples demonstrate the composition and the outstanding performances of this invention.

### Examples

**Example** 1. Synthesis of polyalkoxylated polyester of Formula-2 with different molecular structures. Table 1 lists the preparation of Formula-2 polyalkoxylated polyols polyesters with a range of molecular weight of alkoxylated sorbitol and fatty acids. The reaction flask used is a one liter resin kettle with four necks. The sorbitol (polyols) was mixed with KOH or NaOH base catalyst, and dried at 90°C to 110°C under vacuum. X moles of ethylene oxide or blend of ethylene oxide and propylene oxide per mole of sorbitol are added under pressure and reacted at 140°C to 180°C over the course of 10-20 hours reaction time. After the reaction, the resulting product is cooled down, degassed, and filtered to obtain Sorbeth-xxx. The ingredients of Sorbeth-xxx, oleic acid or stearic acid, and the catalyst - methanesulfonic acid were added into the flask, followed by purging with nitrogen gas. The mixture was heated to between 120°C to 220°C while mixing under the nitrogen and collecting the water. The reaction continued until the sum of R₁ (or acid value) reached the target or near constant. The products were collected after cooling the flak down to room temperatures. The resulting polyesters were waxy solid.

| **Table 1. Preparation of the Range of Polyester thickener of Formula-2** | | | | | | | |
|---|---|---|---|---|---|---|---|
| | **Polyester-1** | **Polyester-2** | **Polyester-3** | **Polyester-4** | **Polyester-5** | **Polyester-6** | **Polyester-7** |
| Sorbeth-160 | 80.5% | | | | | | |
| Sorbeth-170 | | 81.4% | | | | | |
| Sorbeth-230 | | | 87.6% | 87% | 86.9% | | |
| Sorbeth-300 | | | | | | 88.8% | 88.1% |
| Isostearic acid | 19.1% | | | | | | |
| Oleic acid | | | 11.8% | | | | |
| Stearic Acid | | 18.2% | | 12.6% | 12.7% | 10.8% | 11.5% |
| 70% methanesulfonic acid | 0.4% | 0.4% | 0.4% | 0.4% | 0.4% | 0.4% | 0.4% |

**Example 2.** The easy-to-use liquid thickeners of the embodiments of this invention are prepared by mixing the above polyalkoxylated polyols polyesters of Formula-2 with the appropriate solvents or carriers and water. Table 2 shows some of the non-limiting examples.

| **Table2.Preparationof theliquidthickeners of thisinvention-1** | | | | | | | |
|---|---|---|---|---|---|---|---|
| | LT-1 | LT-2 | LT-3 | LT-4 | LT-5 | LT-6 | LT-7 |
| Polyester-3, #26 | | 55% | | | | | 50% |
| Polyester-4, #29 | 50% | | | | | | |
| Polyester-5, # 38 | | | 60% | | | | |
| Polyester-6, # 16 | | | | 50% | | | |
| Polyester-7, #11 | | | | | 50% | 50% | |
| Water | 25% | 26% | 20% | 25% | 25% | 25% | 25% |
| Decyl glucoside | | 13% | 12% | | 25% | | |
| Polysorbate 20 | | | | | | | |
| Polysorbate 80 | | | | | | 25% | |
| Sorbitan caprylate/caprate | 25% | | | | | | |
| Sorbitan Laurate | | 6% | | | | | |
| PEG-7 Glyceryl caprylate/caprate | | | 8% | | | | |
| Capryloyl/Caproyl Methyl Glucamide | | | | | | | 25% |
| PEG-7 Glyceryl cocoate | | | | 25% | | | |

**Example 3.** The liquid thickener examples in Table 2 can thicken both of non-sulfate and sulfate surfactant cleansing products. To demonstrate their thickening efficiency, LT-3 liquid thickener was compared with the commercial liquid thickeners of prior arts in a non-sulfate surfactant cleansing formulation, comprising of x% of the liquid thickeners, 8.67% of Sodium cocoyl Glutamate (Amisoft CS-11, supplied by Ajinomoto Inc.), 1% of cocamide MEA, 0.3% of EDTA, 1 % of NaCl, and Q.S. of water and citric acid to pH 7, perfume, preservative. Sources of commercial thickeners: Crothix Liquid with INCI name of PEG-150 Pentaerythrityl Tetrastearate (and) PEG-6 Caprylic/Capric Glycerides (and) water, and Versathix with INCI name of PEG-150 Pentaerythrityl Tetrastealrate (and) PPG-2 Hyroxyethyl Cocamide (and) water are manufactured by Croda Inc. Glutamate VLT are manufactured by Lubrizol with INCI name of PEG-120 Methyl Glucose Trioleate (and) Propylene Glycol (and) water. Arlypon TT is manufactured by BASF with INCI name of PEG-PPG 120/10-Trimethlolpropane Trioleate (and) Laureth-2.

| **Table 3. Superior thickening of the liquid thickener of this invention** | | | | | |
|---|---|---|---|---|---|
| Thickener | **LT-3** | **CrothixLiquid** | **Versathix** | **GlutamateVLT** | **Arlypon TT** |
| % X to 1000 cP | 2.6 % | 3.8% | 3.3% | 3.5% | 3.0% |

The sodium cocoyl glutamate surfactant is derived from glutamic acid amino acid. It is a well-known mild surfactant. It is also known to be very difficult to thicken; as evidenced by the low thickening performance by the well-known commercial thickeners - Crothix liquid, Versathix, Glutamate VLT, and Arlypon TT in Table 3. In contrast, LT-3 of this invention showed more than 3 times thickening efficacy than all of these commercial thickeners.
**Example 4.** Table 4 shows other non-limiting examples of the liquid thickeners of this invention comprising of the Formual-2 polyalkoxylated polyols polyesters and the appropriate solvents and water.

| **Table4.Preparation of theliquidthickenersofthis invention-2** | | | | | |
|---|---|---|---|---|---|
| | **LT-8** | **LT-9** | **LT-10** | **LT-11** | **LT-12** |
| Polyester-3 | 55% | 55% | 60% | 55% | 55% |
| | | | | | |
| Water | 23% | 22% | 20% | 23% | 23% |
| Cocamide DIPA | | | 14% | 22% | |
| Lauramide DIPA | 22% | 20% | | | |
| Mysistamide DIPA | | | | | 22% |
| Cocamide MEA | | 3% | | | |
| Decyl Glucoside | | | 6% | | |
| Polysorbate-20 | | | | | |
| | | | | | |
| Cocamide DIPA, Lauramide DIPA, Mysistamide DIPA, and Cocamide MEA with trade names of ColaLiquid DC, DL, DM, and ColaMid CMA, respectively are supplied by Colonial Chemical Inc. Decyl Glucoside with trade name of Plantaren 2000NUP, supplied by BASF. | | | | | |

**Example 5.** Several of the liquid thickeners of this invention in Table 4 were formulated into a personal cleansing products based on the extremely mild amino-acid derived surfactant, Sodium Cocoyl Glutamate, with trade name of Amisoft CS-11, supplied by Ajinomot Inc. The composition, by weight of this cleansing product, comprises of 77.23% of water, 8.67% of Amisoft CS-11, 8.0% of Cocamidopropyl Betaine (trade name Monateric LMAB, supplied by Mona Industries), 4% of cocamidopropyl hydroxysultaine (trade name Cola Teric CBS-HP, supplied by Colonial Chemicals), 1% Glyceryl Monolaurate, 0.1% of EDTA, 1% of NaCl, and Q.S. of perfume oils, preservative, etc.

| **Table5.Thickening performanceof theliquidthickeners of thisinvention** | | | |
|---|---|---|---|
| Liquid thickener # | **LT-11** | **LT-8** | **LT-12** |
| % in the formulation | 3.2% | 3.2% | 3.2% |
| Viscosity, cP | 6770 | 8670 | 7040 |

**Example 6.** This example demonstrated the superior performance of the liquid thickeners of embodiments of this invention over the commercial thickener in the classical sulfate surfactant product, which comprises of 10.7% of sodium lauryl ether sulfate (70% solution), 8.58 % of cocamidopropyl betaine (35% solution), 0.25% Cocamide MEA, 0.2% EDTA, 0.5% salt, x% of thickener, Q.S. of water and citric acid to pH 5.5. LT-1 of Table 2 at 0.31% and Versathix at 0.3% in the surfactant formulation gave the viscosity of 16140 cP and 4080 cP, respectively. LT-1 of this invention has a more than six times thickening performance than that of Versathix.
**Example 7.** This example demonstrates the application of the liquid thickener of this invention in a topical skin care application. A daily skin lotion was prepared at 65°C to 80°C, comprising by weight of a) Phase A: 10% caprylic/capric triglyceride, 10% Dimethicone oil, and 1.5% of cetearyl alcohol & Cetearyl glucoside (Trade name: Montanov 68, an emulsifier, supplied by Seppic Inc.); and b) phase B: 3% of LT-2, 3% glycerine, 0.1% EDTA, 0.3% preservative. The final lotion has a pH of 5.8 and viscosity of 13520 cP at 5 rpm.
**Example 8.** Intensive hair cuticle smoothing mask for damaged hair after hair coloring, perm, relaxing, straightening, etc. This hair treatment product is comprised of by weight of a) Phase A: 89.05% of water, 3% of glycerin, 0.1% of EDTA; b) 1.8% of Behentrimonium Chloride, 3% cetearyl alcohol, 0.8% of cetereth-20, 1 % of Argon oil, 1% of LT-2; and c) Q.S. of preservatives and citric acid to pH 5.5. The phase B was prepared by mixing all ingredients of phase B at 75°C to 85°C with a propeller. It was added to Phase A while mixing at 600- 800 rpm and 75°C to 85°C. After cooling down to room temperature, add Phase C. The final product is smooth and shear-thinning with a viscosity of 16800 cP at 5 rpm.
**Example 9.** Table 6 illustrates the potential surfactant formulations of embodiments of this invention with skin/hair actives and other additives.

| **Table6.Prototype formulationsof Surfactant CleansingProducts** | | | | | |
|---|---|---|---|---|---|
| Trade Name | INCI name | #1 | #2 | #3 | #4 |
| | Water | Q.S. to 100% | Q.S. to 100% | Q.S. to 100% | Q.S. to 100% |
| Amisoft CS-11 | Sodium Cocoyl Glutamate | | 8% | | |
| Iselux Flake | Sodium Lauroyl methyl Isethionate | 10% | | | |
| Bioterge-AS-90 Bead | Sodium C14-16 Olefin sulfonate | | | 8% | 8% |
| Jaguar Excel | Guar Hydroxypropyltrimonium Chloride | 0.4% | | | |
| Jaguar C-13S | Guar Hydroxypropyltrimonium Chloride | | 0.4% | | |
| Ucare JR-400 | Polyquaternium-10 | | | 0.5% | 0.5% |
| Monateric LMAB | Cocamidopropyl Betaine | 10% | 8% | 10% | 10% |
| ColaTeric CBS-HP | Cocamidopropyl Hydroxysultaine | | 8% | | |
| Plantaren 2000N UP | Decyl glucoside | | | 5% | |
| | LT-2 of Table 2 | 1.0% | | | |
| | LT-3 of Table 2 | | 2.0% | | |
| | LT-8 of Table 3 | | | 0.5% | 0.5% |
| | Glyceryl Monolaurate | 1.0%% | 1.0% | 1.0% | |
| Protamide CME | Cocamide MEA | | | | 1.0% |
| DC 5-7113 | Silicone quaternium-16 (and) Undeceth-11 (and) Butylocanol (and) Undeceth-5 | 1.0% | 1.0% | 1.0% | 1.0% |
| | NaCL | | 1.0% | 1.0% | 1.0% |
| Versene 100 | Tetra sodium Ethylenediaminetetraacetate | 0.1% | 0.1% | 0.1% | 0.1% |
| | Preservative | Q.S. | Q.S. | Q.S. | Q.S. |
| | Perfume oil | Q.S. | Q.S. | Q.S. | Q.S. |
| | Citric acid Q.S. to pH | 5.5 | 5 | 5.5 | 5.5 |

**Example 10.** Table 7 of this example illustrates the synthesis of Formula-1 Polyester with the twin-branched Guerbet acid. The commercial Guerbet acid used are Isocarb-20 (2-octyl-dodecanoic acid), having 20 carbons and Isocarb-24 (2-decyl-tetradecanoic acid), having 24 carbons, supplied by Sasol. The same reaction conditions of example 1 is applied here.

| Table 7. Synthesis of Formula-2 polyester derived from Guerbet Acid | | |
|---|---|---|
| | **Polyester-10** | **Polyester-11** |
| Sorbeth-230 | 82.50% | 82.68% |
| Isocarb-20 | 16.85% | |
| Isocarb-24 | | 6.04% |
| Oleic acid | | 10.94% |
| Methane Sulfonic Acid (70%) | 0.65% | 0.65% |

**Example 11.** This example shows the non-limiting examples of liquid thickener composition comprising the polyesters of example 10. Table 8 illustrates the potential liquid thickener compositions comprising of polyester-10 and polyester-11.

| **Table 8. Liquid thickeners made of Polyesters of Gurbet Acid** | | | |
|---|---|---|---|
| Liquid thickener # | **LT-13** | **LT-14** | **LT-16** |
| Polyeter-10 | 55% | | |
| Polyester-11 | | 55% | 50% |
| Cocamide DIPA | 20% | | |
| Lauramide DIPA | | 20% | |
| Decyl glucoside | | 5% | 15% |
| Polysorbate 20 | 5% | | |
| Sorbitan Laurate | | | 10% |
| | | | |
| water | 20% | 20% | 25% |

**Example 12.** This example illustrates the amazing thickening power of the above liquid thickeners in comparison to the commercial product - Crothix Liquid. Table 9 shows the compositions of two common prototype shampoo bases. Table 10 shows the viscosities of the shampoo prototypes with the liquid thickeners of this invention of Table 8, and the Crothix Liquid. Note in Table 10 that LT14 at 3% exhibited about 37 times thickening power than Corthix Liquid at 4.5%.

| Table 9. Common Shampoo Prototype Bases | | |
|---|---|---|
| | **SH-1** | **SH-2** |
| Water | 78.9% | 58.4% |
| Sodium C14-C16 Olefin Sulfonate (39%) | | 30% |
| Potassium Cocoyl Glycinate powder | 6% | |
| Cocamidopropyl betaine | 15% | 11.5% |
| LDTA chelator | 0.1% | 0.1% |
| Citric acid | q.s. to pH 7 | q.s. to pH 5.5 |

| Table 10. Thickening Performance of Liquid Thickeners in Shampoo Prototypes | | | | | | |
|---|---|---|---|---|---|---|
| | SH1-Cl | SH1-LT16 | SH1-LT14 | SH2-Cl | SH2-LT16 | SH2-LT14 |
| SH-1 | 95.5% | 96.5% | 96.5% | | | |
| SH-2 | | | | 95.5% | 97% | 97% |
| Crothix Liquid | 4.5% | | | 4.5% | | |
| LT-16 | | 3.5% | | | 3.0% | |
| LT-14 | | | 3.5% | | | 3.0% |
| Viscosity, cP | 1702 | 37440 | 29800 | 913 | 14380 | 34120 |

## Claims

1. A liquid thickener composition comprising:
20%-80% of the polyalkoxylated polyols polyester of the formula:
Q-[(OA)ₙ-OR]ₘ Formula-2
Q is a radical of organic polyol compounds, having 6 to 50 carbon atoms, and is saturated or unsaturated, straight, branched or cyclic chain structure, wherein one or more of said carbon atoms are independently substituted with a substituent having the formula of [(OA)ₙ₋OR], wherein n = 6 to 25,
wherein each of the remaining carbon atoms of the 6 to 50 carbon atoms are independently substituted with hydrogen, oxygen, or nitrogen,
A is selected from -C₂H₄- or -C₃H₆-,
R is independently selected from hydrogen or -COR₁,
wherein the averaged total number of COR₁ in the composition is ≥2,
R₁ is independently selected from C₆-C₃₄-alkyl, preferably derived from a Guerbet acid; C₆-C₂₂-hydroxyalkyl, preferably derived from a Guerbet acid; C₆ - C₂₂ - alkenyl, preferably derived from a Guerbet acid;
wherein R₁ is preferably derived from stearic, isostearic, oleic acid, Guerbet acid, or mixtures thereof,
n is an integer independently selected from 1-125, and
m is an integer selected from 6-25;
an organic carrier, selected from the group consisting of: organic solvents, hydrophobic thickeners, or mixtures thereof,
the organic solvents are selected from the group consisting of: mono- or polyhydric alcohols and their ether, ester, or amide derivatives, glycerol, propylene glycol, 1.3-propanediol, butylene glycol, dipropylene glycol, pentylene glycol, methoxy diglycol, polyglyceryl-3, polyglyceryl-2, glycereth-7, glycereth-26, diglycerol, sorbitol, glucose, methyl glucose, methyl glucamide, glyceryl laurate, glyceryl oleate, glyceryl isostearte, propylene glycol monolaurate, PEG-6 caprylic/capric glyceride, sorbitan carpylate/caprate, sorbitan sesquicaprylate, sorbitane laurate, methyl glucose caprate/caprylate/oleate, PEG-7 glyceryl cocoate, fatty methyl ester ethoxylates, alkylpolyglucosides, decyl glucoside, cocoa glucoside, polysorbate 20, polysorbate 60, and polysorbate 80, alkyoyl methyl glucamide, lauroyl methyl glucamide, capryloyl/caproyl methyl glucamide, and
the hydrophobic thickeners are selected from the group consisting of: non-ionic alkanolamides, liquid alkanolamides, ethoxylated C₁₀ - C₁₄ alcohols, Laureth-1 to Laureth-5, dimethyl lauramide/myristamide, PPG-2 Hydroxyethyl Cocamide, Cocamide DIPA, lauramide DIPA, soyamide DIPA; and
water, wherein the ratio of water to organic solvent carrier by weight is from 4:1 to 1:4.

2. The liquid thickener composition of claim 1, wherein the C₆-C₃₄-alkyl derived from a Guerbet acid has the following formula: 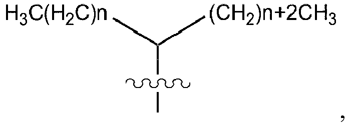 wherein n = 1-120, preferably 3, 5, 7, 9, or 13.

3. The liquid thickener composition of claim 1 or 2, wherein the liquid thickener composition further comprises:
20-80% of the polyalkoxylated polyol polyester of the Formula-2, wherein the Q is a radical of the following polyols compounds:
sugar alcohols, polyhydric alcohol, or polyalcohol with at least six hydroxyl groups, having the general formula of HOCH₂ (CHOH)ₙ CH₂OH, mannitol, sorbitol, galactitol, fucitol, iditol, inositol, volemitol, isomalt, maltitol, lactitol, maltotritol, and maltotetraitol;
disaccharide having a glycosidic linkage,
di-pentaerythritol,
dextrin with a chemical structure of (C₆H₁₀O₅)ₙ, where n is from 2 to 20,
dendrimer polyols, and
polyglyceryls with 3 to 10 glycerin units and 6 or more hydroxyl groups;
an organic carrier, selected from the group consisting of: organic solvents, hydrophobic thickener, or mixtures thereof,
the organic solvents are selected from the group consisting of: mono- or polyhydric alcohols and their ether, ester, or amide derivatives, glycerol, propylene glycol, 1.3-propanediol, butylene glycol, dipropylene glycol, pentylene glycol, methoxy diglycol, polyglyceryl-3, polyglyceryl-2, glycereth-7, glycereth-26, diglycerol, sorbitol, glucose, methyl glucose, methyl glucamide, glyceryl laurate, glyceryl oleate, glyceryl isostearte, propylene glycol monolaurate, PEG-6 caprylic/capric glyceride, sorbitan carpylate/caprate, sorbitan sesquicaprylate, sorbitane laurate, methyl glucose caprate/caprylate/oleate, PEG-7 glyceryl cocoate, fatty methyl ester ethoxylates, alkylpolyglucosides, decyl glucoside, cocoa glucoside, polysorbate 20, polysorbate 60, and polysorbate 80, alkyoyl methyl glucamide, lauroyl methyl glucamide, capryloyl/caproyl methyl glucamide, and
the hydrophobic thickeners are selected from the group consisting of: non-ionic alkanolamides, liquid alkanolamides, ethoxylated C₁₀ - C₁₄ alcohols, Laureth-1 to Laureth-5, dimethyl lauramide/myristamide, PPG-2 Hydroxyethyl Cocamide, Cocamide DIPA, lauramide DIPA, soyamide DIPA; and
water, wherein the ratio of water to organic solvent carrier by weight is from 4:1 to 1:4.

4. A liquid thickener composition of claim 1 or 2, wherein the liquid thickener composition further comprises:
20-80% of the polyalkoxylated polyols polyester of the Formula-2, wherein the Q is the radical of the following polyols compounds:
sugar alcohols, polyhydric alcohol, or polyalcohol with at least six hydroxyl groups, having the general formula of HOCH₂ (CHOH)ₙ CH₂OH, mannitol, sorbitol, galactitol, fucitol, iditol, inositol, volemitol, isomalt, maltitol, lactitol, maltotritol, maltotetraitol, and polyglycitol,
disaccharide, which is formed from two monosaccharides by dehydration via glycosidic linkage,
di-pentaerythritol,
dextrin with a chemical structure of (C₆H₁₀O₅)ₙ, where n is from 2 to 20, and
dendrimer polyols;
the organic carrier is selected from the group consisting of: organic solvents, hydrophobic thickeners, or mixtures thereof,
the organic solvents are selected from the group consisting of: mono- or polyhydric alcohols and their ether, ester, or amide derivatives, glycerol, propylene glycol, 1.3-propanediol, butylene glycol, dipropylene glycol, pentylene glycol, methoxy diglycol, polyglyceryl-3, polyglyceryl-2, glycereth-7, glycereth-26, diglycerol, sorbitol, glucose, methyl glucose, methyl glucamide; glyceryl laurate, glyceryl oleate, glyceryl isostearte, propylene glycol monolaurate, PEG-6 caprylic/capric glyceride, sorbitan carpylate/caprate, sorbitan sesquicaprylate, sorbitane laurate, methyl glucose caprate/caprylate/oleate, PEG-7 glyceryl cocoate, fatty methyl ester ethoxylates, alkylpolyglucosides such as decyl glucoside, cocoa glucoside, polysorbate 20, polysorbate 60, and polysorbate 80, alkyoyl methyl glucamide, lauroyl methyl glucamide, capryloyl/caproyl methyl glucamide, and
the hydrophobic thickeners are selected from the group consisting of: non-ionic alkanolamides, liquid alkanolamides, ethoxylated C₁₀ - C₁₄ alcohols, Laureth-1 to Laureth-5, and dimethyl lauramide/myristamide, PPG-2 Hydroxyethyl Cocamide, Cocamide DIPA, lauramide DIPA, soyamide DIPA; and
water, wherein the ratio of water to organic solvent carrier by weight is from 4:1 to 1:4.

5. A liquid thickener composition of claim 1 or 2, wherein the liquid thickener composition further comprises:
20%-80% of the polyalkoxylated polyols polyester of Formula-2, wherein Q is the radical of sorbitol, trehalose, mannitol, di-pentaerythritol, sucrose, lactose, chitobiose, cellobiose, and maltose;
the organic carrier is selected from the group consisting of: organic solvents, hydrophobic thickeners, or mixtures thereof, wherein
the organic solvents are selected from the groups consisting of: mono- or polyhydric alcohols and their ether, ester, or amide derivatives, glycerol, propylene glycol, 1.3-propanediol, butylene glycol, dipropylene glycol, pentylene glycol, methoxy diglycol, polyglyceryl-3, polyglyceryl-2, glycereth-7, glycereth-26, diglycerol, sorbitol, glucose, methyl glucose, methyl glucamide, glyceryl laurate, glyceryl oleate, glyceryl isostearte, propylene glycol monolaurate, PEG-6 caprylic/capric glyceride, sorbitan carpylate/caprate, sorbitan sesquicaprylate, sorbitane laurate, methyl glucose caprate/caprylate/oleate, PEG-7 glyceryl cocoate, fatty methyl ester ethoxylates, alkylpolyglucosides, decyl glucoside, cocoa glucoside, polysorbate 20, polysorbate 60, and polysorbate 80, alkyoyl methyl glucamide, lauroyl methyl glucamide, and capryloyl/caproyl methyl glucamide, and
the hydrophobic thickeners are selected from the groups consisting of: non-ionic alkanolamides, liquid alkanolamides, ethoxylated C₁₀ - C₁₄ alcohols, Laureth-1 to Laureth-5, dimethyl lauramide/myristamide; PPG-2 Hydroxyethyl Cocamide, Cocamide DIPA, lauramide DIPA, soyamide DIPA; and
water, wherein the ratio of water to organic solvent carrier by weight is from 4:1 to 1:4.

6. A liquid thickener composition of claim 1 or 2, wherein the liquid thickener composition further comprises:
20%-80% of the polyalkoxylated polyols polyester of Formula-2, wherein Q is a radical of sorbitol, trehalose, mannitol, di-pentaerythritol, sucrose, lactose, chitobiose, cellobiose, and maltose;
the organic carrier or mixtures thereof, selected from the group consisting of alkyl polyglucosides, alkyl methyl glucamide, sorbitan laurate, sorbitan caprylate/caprate, liquid non-ionic alkanolamide surfactants, laureth-1, laureth-2, laureth-3, glyceryl caprylate/caprate, and glyceryl laurate; and
water, wherein the ratio of water to organic solvent carrier by weight is from 4:1 to 1:4.

7. A liquid thickener composition of claim 1 or 2, wherein the liquid thickener composition further comprises:
20%-80% of the polyalkoxylated polyols polyester of Formula-2, wherein Q is the radical of sorbitol, trehalose, mannitol, di-pentaerythritol, sucrose, lactose, chitobiose, cellobiose, and maltose;
the organic carrier or mixtures thereof, selected from the groups consisting of the alkanolamides; and
water, wherein the ratio of water to organic solvent carrier by weight is from 4:1 to 1:4.

8. A cosmetic, dermatological, or pharmaceutical composition, in the form of hair cleansing/treatment formulation, skin and body cleansing/treatment formulations, and other toiletry formulations, comprising the liquid thickener composition of any of the preceding claims.

9. A cosmetic, dermatological, or pharmaceutical composition comprising the liquid thickener composition of claim 5, in the form of hair cleansing/treatment formulation, skin and body cleansing/treatment formulations, and other toiletry formulations.

10. A cosmetic, dermatological, or pharmaceutical composition of claim 8 further comprising water, and
1% - 50% by weight of surfactants selected from the group consisting of: anionic surfactant, cationic surfactant, non-ionic surfactant, amphoteric surfactant, and mixtures thereof;
0.1% to 15% of the liquid thickener composition of Formula-2; and
0.1% to 60% of other ingredients selected from the group consisting of: skin and hair actives, stabilizer, further thickeners, and other customary personal cleansing ingredients.

11. A cosmetic, dermatological, or pharmaceutical composition of claim 9, further comprising water, and
1% - 50% by weight of surfactants selected from the group consisting of: anionic surfactant, cationic surfactant, non-ionic surfactant, amphoteric surfactant, and mixtures thereof;
0.1% to 15% of the liquid thickener composition of Formula-1; and
0.1% to 60% of other ingredients selected from the group consisting of: skin and hair actives, stabilizer, further thickeners, and other customary personal cleansing ingredients.

12. A cosmetic, dermatological, or pharmaceutical composition of about 1% to 8% of the liquid thickener composition of any of claims 1-7, further comprising:
about 1% to 25% by weight of a skin and hair active ingredient selected from the group consisting of: UV filters, moisturizers, conditioners, antiseptic agents, deodorant actives, reducing agents for permanent wave products, colorants for coloring hair, anti-aging actives, perfume, petrolatum, vegetable oils, cationic conditioning polymers, and mixtures thereof; and
water.

13. The composition of claim 1 or 2, wherein the Guerbet moiety has 16 to 32 carbons.

14. The composition of claim 1 or 2, wherein the Guerbet moiety has 18 to 24 carbons.

15. The composition of claim 1 or 2, wherein the Guerbet moiety has 20 carbons.
